Europäisches Patentamt

European Patent Office

Office européen des brevets

(19)

(11) Numéro de publication: **0 266 815 B1**

(12) **FASCICULE DE BREVET EUROPEEN**

(45) Date de publication de fascicule du brevet:
06.03.91

(51) Int. Cl.⁵: **C07C 59/01**, C07C 51/09

(21) Numéro de dépôt: **87201935.1**

(22) Date de dépôt: **09.10.87**

(54) **Procédé pour la fabrication de l'acide bêta-hydroxybutyrique et de ses sels par hydrolyse d'oligomères de l'acide bêta-hydroxybutyrique en milieu basique.**

(30) Priorité: **20.10.86 FR 8614627**

(43) Date de publication de la demande:
**11.05.88 Bulletin 88/19**

(45) Mention de la délivrance du brevet:
**06.03.91 Bulletin 91/10**

(84) Etats contractants désignés:
**AT BE CH DE ES FR GB GR IT LI LU NL SE**

(56) Documents cités:
**US-A- 3 044 942**

**CHEMICAL ABSTRACTS, vol. 77, no. 7, 14 août 1972, page 160, résumé 44596t, Columbus, Ohio, US; B. HAUTTECOEUR et al.: "Controlled chemical depolymerization of the poly-beta-hydroxybutyrate lipid from Bacillus megaterium"**

**CHEMICAL ABSTRACTS, vol. 83, no. 17, 27 octobre 1975, page 171, résumé no. 143344q, Columbus, Ohio, US; B. HAUTTECOEUR et al.: "Mild chemical depolymerization of beta-hydroxybutyric lipid (PHB) of Bacillus megaterium. Action of boron trifluoride-methanol mixture on polymers (and oligomers) of d-(-)-3-hydroxybutyric acid"**

(73) Titulaire: **SOLVAY & Cie (Société Anonyme)**
**Rue du Prince Albert, 33**
**B-1050 Bruxelles(BE)**

(72) Inventeur: **Vanlautem, Noel**
**Rue de la Taille, 40**
**B-5790 Jemeppe-sur-Sambre(BE)**
Inventeur: **Coisne, Jean-Marc**
**Boulevard Général Jacques, 182**
**B-1050 Bruxelles(BE)**
Inventeur: **Gilain, Jacques**
**Avenue de la Ramée, 10**
**B-1180 Bruxelles(BE)**

(74) Mandataire: **Lechien, Monique et al**
**Solvay & Cie S.A. Département de la Propriété Industrielle Rue de Ransbeek, 310**
**B-1120 Bruxelles(BE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé pour la fabrication de l'acide bêta-hydroxybutyrique et de ses sels à partir d'oligomères de l'acide bêta-hydroxybutyrique également appelé poly-bêta-hydroxybutyrates comprenant une étape d'hydrolyse basique de ces oligomères.

De nombreux microorganismes sont capables de synthétiser des polymères de l'acide bêta-hydroxybutyrique (poly-bêta-hydroxybutyrates) et diverses techniques, telles que l'extraction, sont connues pour séparer les poly-bêta-hydroxybutyrates des biomasses.

Comme l'acide bêta-hydroxybutyrique est un produit intermédiaire intéressant pour l'industrie chimique fine, diverses tentatives ont été réalisées en vue de l'obtenir au départ de ces polymères obtenus par biosynthèse.

Une méthode connue consiste à saponifier les poly-bêta-hydroxybutyrates au moyen d'une solution alcaline de manière à former des sels alcalins de l'acide bêta-hydroxybutyrique. Cette méthode d'hydrolyse en milieu basique comporte l'inconvénient d'entraîner la formation de sous-produits non désirés tels que l'acide crotonique en quantités importantes (Lemoigne M., C.R. Acad. Sci. Paris 1924, 178, p. 1093-1095/CA 18-1940; Lemoigne M., Bull. Soc. Chim. Biol. 1926, 8, p. 770-782; HAUTTE COEUR, B. et al. C.R. Acad. Sci. Paris. Ser.D.1972, 274 (19), 2729-2732).

Une autre méthode consistant en une hydrolyse acide en milieu organique (brevet FR 2.486.072) donne des bons résultats du point de vue de la pureté et des rendements des produits obtenus mais nécessite un équipement industriel plus complexe et une dépense en énergie relativement élevée.

On a maintenant trouvé un procédé qui ne présente plus les inconvénients des procédés connus et qui permet d'éviter des volumes réactionnels importants ainsi que la dépense énergétique de l'évaporation de l'eau. Ce procédé, qui de plus est rapide, mène à une solution d'acide bêta-hydroxybutyrique dont la teneur en dimère est faible et n'entraîne pas de formation subséquente d'acide crotonique et/ou d'oligomères insaturés. Ce procédé permet en outre d'obtenir de l'acide bêta-hydroxybutyrique de grande pureté et sous forme directement cristallisable.

La présente invention concerne à cet effet un procédé pour la fabrication de l'acide bêta-hydroxybutyrique et de ses sels à partir d'oligomères de l'acide bêta-hydroxybutyrique, dans lequel on soumet les oligomères à une hydrolyse dans un mélange réactionnel liquide contenant une base et un solvant des oligomères de l'acide bêta-hydroxybutyrique qui est stable chimiquement vis-à-vis de la base mise en oeuvre.

Les oligomères peuvent être obtenus par tout moyen. Ils proviennent généralement de l'hydrolyse acide de poly-bêta-hydroxybutyrates, obtenus à partir d'une biomasse par tout moyen de séparation connu.

L'origine de la biomasse est sans importance pour le procédé selon l'invention. La biomasse peut provenir de divers microorganismes et notamment de bactéries comme il a été décrit dans Angewandte Chemie, 1962, 74ème année, N 10, pages 342 à 346 par Schlegel et Gottschalk.

De préférence, les oligomères, mis en oeuvre dans le procédé selon l'invention, ont une masse moléculaire moyenne en nombre ($\overline{M}$ n) inférieure à 500 daltons. Tout particulièrement préférés sont les oligomères ayant une masse moléculaire moyenne inférieure à 250 daltons solubles dans le solvant sous forme acide ou sous forme salifiée.

De préférence, on utilise des oligomères provenant de l'hydrolyse acide de poly-bêta-hydroxybutyrates telle que décrite dans le brevet français 2.486.072 déposé le 3.7.80 au nom de Solvay & Cie. Cette hydrolyse qui a lieu en présence d'un catalyseur acide et d'un solvant organique, peut être arrêtée à tout moment après que des oligomères, tels que spécifiés ci-dess ont été obtenus, en vue d'être suivie du procédé selon l'invention. Généralement l'hydrolyse acide des poly-bêta-hydroxybutyrates est arrêtée au moment où l'hydrolyse acide pourrait être poursuivie dans une phase liquide aqueuse et que le solvant a été éliminé en partie par distillation. De préférence, l'hydrolyse acide est arrêtée lorsque les oligomères obtenus sont solubles dans l'eau. A ce stade, la masse moléculaire moyenne ($\overline{M}$ n) des oligomères obtenus est comprise entre 110 et 200 daltons.

Le solvant mis en oeuvre dans le procédé selon l'invention peut être n'importe quelle substance qui permet au moins partiellement de disperser et de solubiliser les oligomères de l'acide bêta-hydroxybutyrique dans les conditions basiques du procédé. Habituellement le solvant est polaire et a une température d'ébullition à pression atmosphérique inférieure à 160° C. De préférence on met en oeuvre un alcool, une amide, un éther ou de l'eau, particulièrement préférés sont les solvants tels que l'eau, le dioxanne, l'éthanol, le méthanol et le tétrahydrofuranne. Le solvant tout particulièrement préféré est l'eau.

La base mise en oeuvre dans le procédé selon l'invention peut être n'importe quelle base minérale ou organique. Les bases généralement utilisées sont des oxydes ou des hydroxydes solubles dans le solvant des oligomères de l'acide bêta-hydroxybutyrique mis en oeuvre. Habituellement, on met en oeuvre une base forte, telle qu'un hydroxyde d'un métal alcalin ou alcalino-terreux. Parmi ceux-ci, on travaille, de préférence, avec l'hydroxyde de calcium, l'hydroxyde de sodium ou l'hydroxyde de potassium.

L'addition de la base peut être continue ou discontinue. La base ajoutée sert dans un premier temps à neutraliser l'acidité existante et dans un deuxième temps à effectuer la saponification. Une manière de contrôler cette réaction de saponification peut être par exemple de se maintenir à un pH donné (compris entre 7 et 14 et de préférence entre 9 et 12; des valeurs de pH ayant donné de bons résultats sont comprises entre 10 et 11) par addition contrôlée de la base. La réaction est considérée comme terminée lorsque le pH est stable sans addition de base.

Habituellement, on opère à des températures supérieures à 25°C. En général, pour des raisons de commodité et de stabilité thermique des oligomères de l'acide bêta-hydroxybutyrique ou des sels de l'acide bêta-hydroxybutyrique, on n'opère pas à des températures supérieures à 150°C. De bons résultats ont été obtenus à des températures situées entre 40 et 100°C et de préférence entre 50 et 80°C.

La pression appliquée au cours du procédé est généralement comprise entre 0,1 et 10 bars et de préférence elle est égale à la pression atmosphérique.

Dans le procédé selon l'invention, le mélange réactionnel peut être constitué d'une phase ou de plusieurs phases distinctes. On préfère toutefois travailler avec un mélange réactionnel où les oligomères, la base et l'eau ne constituent qu'une seule phase liquide homogène. On opère donc de préférence en l'absence d'une seconde phase liquide ou d'une phase solide comportant des oligomères de l'acide bêta-hydroxybutyrique non dissous.

La durée de l'hydrolyse est déterminée cas par cas, elle est fonction du degré de polymérisation des oligomères de départ.

A la fin du procédé d'hydrolyse, on obtient une solution contenant le sel de l'acide bêta-hydroxybutyrique et de la base mise en oeuvre dans le milieu réactionnel.

Dans le cas où l'on souhaite obtenir ce sel, il peut être purifié par tout moyen connu tel que la cristallisation.

Dans le cas où l'on souhaite obtenir l'acide bêta-hydroxybutyrique, on ajoute à la fin de l'hydrolyse basique un acide dans le milieu réactionnel aqueux. Pour cela, n'importe quel acide plus fort que l'acide bêta-hydroxybutyrique convient. Habituellement, et pour des raisons pratiques de purification, on utilise l'acide chlorhydrique. L'acide bêta-hydroxybutyrique obtenu peut ensuite être séparé du milieu par tout moyen connu. Par exemple, on peut éliminer l'eau par évaporation et filtrer en vue d'éliminer le sel formé. Cette séparation peut également être effectuée par une extraction à l'aide d'un solvant de l'acide bêta-hydroxybutyrique, tel que l'éther ou l'acétate d'éthyle; ce solvant étant ensuite éliminé par évaporation.

L'acide bêta-hydroxybutyrique se présente sous la forme d'une masse cristalline incolore qui peut éventuellement être ensuite purifié par tout moyen connu, par exemple, par recristallisation ou par fusion de zone.

L'acide bêta-hydroxybutyrique et ses sels obtenus selon le procédé de l'invention peuvent être utilisés dans toutes les applications connues de ces produits, c'est-à-dire notamment comme médicament, comme produit intermédiaire dans l'industrie chimique fine ou comme additif dans la nourriture animale.

Les exemples qui suivent servent à illustrer l'invention sans toutefois en limiter la portée.

Exemple 1 (comparatif)

Dans un ballon de 500 cm³ muni d'un agitateur, d'un thermomètre, d'une sonde de pH, d'un réfrigérant et d'une ampoule d'introduction, on introduit 10 g de poly-bêta-hydroxybutyrate dont la masse moléculaire moyenne en nombre ($\overline{M}$ n) est égale à $0,23.10^6$, ainsi que 10 ml d'eau et 90 ml de dioxanne en vue de solubiliser le poly-bêta-hydroxybutyrate.

Le mélange réactionnel est chauffé à 85°C sous agitation jusqu'à ce que tout le poly-bêta-hydroxybutyrate soit dissous et la température est ensuite ramenée à 70°C.

On ajoute ensuite lentement une solution d'hydroxyde de sodium 1 N au mélange réactionnel jusqu'à obtention d'un pH compris entre 10,5 et 11 que l'on maintient par addition contrôlée de la solution d'hydroxyde de sodium.

La réaction est arrêtée après 3,5 heures lorque le pH est stable sans addition d'hydroxyde de sodium (volume introduit 120 ml) et le mélange réactionnel est refroidi.

L'analyse de ce mélange montre que l'acide bêta-hydroxybutyrique est formé avec un rendement de 46 % vis-à-vis du poly-bêta-hydroxybutyrate mis en oeuvre, le mélange réactionnel contient également de l'acide crotonique (45 %). L'acide crotonique représente donc 50 % molaire de l'ensemble acide bêta-hydroxybutyrique + acide crotonique.

Exemple 2 (selon l'invention)

On place dans un réacteur 260 ml d'une solution organique de 1,2-dichloroéthane (1,2-DCE ₐ) contenant 68 g d'oligomères de l'acide bêta-hydroxybutyrique de masse moléculaire moyenne en nombre ($\overline{M}_n$) égale à 270, obtenue selon l'exemple 6 du brevet français 2.486.072.

Le réacteur est équipé de manière telle que l'on puisse éliminer le solvant organique pendant que l'on y ajoute graduellement de l'eau. Lorsque la totalité de solvant organique a été éliminée, on obtient une solution aqueuse (175 ml) d'oligomères de l'acide poly-$\beta$-hydroxybutyrique de $\overline{M}_n$ égale à 120-130.

Cette solution est décolorée par traitement par du charbon actif et l'acide p-toluènesulfonique est éliminé par passage sur une colonne échangeuse d'anions préalablement conditionnée sous forme bêta-hydroxybutyrate.

La solution résultante est introduite dans un ballon de 500 cm³ muni d'un agitateur mécanique, d'un thermomètre, d'un réfrigérant à eau, d'une sonde de pH et d'une ampoule d'introduction contenant une suspension aqueuse d'hydroxyde de calcium [185 g Ca(OH)₂ par litre].

La suspension d'hydroxyde de calcium est additionnée lentement à température ambiante.

Lorsque le pH est égal à 10,5, la température du mélange réactionnel atteint spontanément 45° C. La température est amenée par chauffage à 70° C, on maintient le pH à 10,5 par addition contrôlée de la suspension d'hydroxyde de calcium.

Durant l'hydrolyse, le ballon est maintenu sous légère pression d'azote.

L'hydrolyse dure environ 2 ,5 heures.

Lorsque le pH se maintient au cours du temps à 10,5 sans addition de suspension d'hydroxyde de calcium, l'hydrolyse est arrêtée.

Le mélange réactionnel est alors refroidi à température ambiante et est ensuite acidifié à pH 1 par addition d'une solution d'acide chlorhydrique 10 N.

La solution résultante est concentrée par évaporation à une température de l'ordre de 50° C et sous une pression de 25 mbars jusqu'à ce que la teneur totale en eau soit égale à 60 %.

La solution aqueuse obtenue est ensuite soumise à des extractions répétées par de l'acétate d'éthyle. Puis, le solvant est évaporé.

On obtient ainsi l'acide bêta-hydroxybutyrique avec un rendement molaire égal à 87 % vis-à-vis du poly-bêta-hydroxybutyrate mis en oeuvre.

La proportion en acide crotonique dans le produit ainsi isolé est égale à 1,6 % molaire de l'ensemble acide bêta-hydroxybutyrique + acide crotonique.

## Revendications

1. Procédé pour la fabrication de l'acide bêta-hydroxybutyrique et de ses sels comprenant une réaction d'hydrolyse par voie basique caractérisé en ce que l'on soumet des oligomères de l'acide bêta-hydroxybutyrique à une hydrolyse dans un mélange réactionnel liquide contenant une base et un solvant des oligomères de l'acide bêta-hydroxybutyrique qui est chimiquement inerte vis-à-vis de la base mise en oeuvre.

2. Procédé selon la revendication 1 caractérisé en ce que les oligomères mis en oeuvre ont une masse moléculaire inférieure à 500 daltons.

3. Procédé selon la revendication 1 ou 2 caractérisé en ce que les oligomères mis en oeuvre sont solubles dans le solvant sous forme salifiée ou sous forme acide.

4. Procédé selon l'une quelconque des revendications 1 à 3 caractérisé en ce que l'on met en oeuvre un solvant dans lequel la base est soluble.

5. Procédé selon l'une quelconque des revendications 1 à 4 caractérisé en ce que le solvant mis en oeuvre est de l'eau.

6. Procédé selon l'une quelconque des revendications 1 à 5 caractérisé en ce que l'on met en oeuvre une base forte.

7. Procédé selon la revendication 6 caractérisé en ce que l'on met en oeuvre un hydroxyde d'un métal alcalin ou alcalinoterreux.

8. Procédé selon la revendication 7 caractérisé en ce que l'on met en oeuvre une base choisie parmi l'hydroxyde de calcium, l'hydroxyde de sodium ou l'hydroxyde de potassium.

9. Procédé selon l'une quelconque des revendications 1 à 8 caractérisé en ce que le mélange réactionnel est maintenu à un pH compris entre 9 et 12.

10. Procédé selon l'une quelconque des revendications 1 à 9 caractérisé en ce que le mélange réactionnel est maintenu à une température comprise entre 50 et 80° C.

11. Procédé pour la fabrication de l'acide bêta-hydroxybutyrique et de ses sels à partir de polymères, caractérisé en ce qu'il comprend au moins les étapes suivantes :

a) dans une première étape, on soumet les polymères à une hydrolyse dans un mélange réactionnel liquide contenant un solvant organique, ces polymères, un catalyseur acide et de l'eau, jusqu'à l'obtention d'oligomères.

b) dans une deuxième étape, on soumet les oligomères obtenus à la première étape à une hydrolyse dans un mélange réactionnel liquide contenant une base et un solvant des oligomères de l'acide bêta-hydroxybutyrique qui est chimiquement inerte vis-à-vis de la base mise en oeuvre.

## Claims

1. Process for the manufacture of beta-hydroxybutyric acid and of its salts comprising a basic hydrolysis reaction, characterized in that oligomers of beta-hydroxybutyric acid are subjected to a hydrolysis in a liquid reaction mixture containing a base and a solvent for the oligomers of beta-hydroxybutyric acid, which is chemically inert towards the base employed.

2. Process according to Claim 1, characterized in that the oligomers employed have a molecular mass below 500 daltons.

3. Process according to Claim 1 or 2, characterized in that the oligomers employed are soluble in the solvent in salt form or in acidic form.

4. Process according to any one of Claims 1 to 3, characterized in that a solvent in which the base is soluble is employed.

5. Process according to any one of Claims 1 to 4, characterized in that the solvent employed is water.

6. Process according to any one of Claims 1 to 5, characterized in that a strong base is employed.

7. Process according to Claim 6, characterized in that an alkali metal or alkaline-earth metal hydroxide is employed.

8. Process according to Claim 7, characterized in that a base chosen from calcium hydroxide, sodium hydroxide or potassium hydroxide is employed.

9. Process according to any one of Claims 1 to 8, characterized in that the reaction mixture is maintained at a pH of between 9 and 12.

10. Process according to any one of Claims 1 to 9, characterized in that the reaction mixture is maintained at a temperature of between 50 and 80° C.

11. Process for the manufacture of beta-hydroxybutyric acid and of its salts from polymers, characterized in that it comprises at least the following stages:

a) in a first stage, the polymers are subjected to a hydrolysis in a liquid reaction mixture containing an organic solvent, these polymers, an acidic catalyst and water until oligomers are obtained;

b) in a second stage, the oligomers obtained at the first stage are subjected to a hydrolysis in a liquid reaction mixture containing a base and a solvent for the oligomers of beta-hydroxybutyric acid, which is chemically inert towards the base employed.

## Ansprüche

1. Verfahren zur Herstellung von beta-Hydroxybuttersäure und ihren Salzen, das eine Hydrolysereaktion auf basischem Weg umfaßt, dadurch gekennzeichnet, daß man Oligomere von betaHydroxybuttersäure einer Hydrolyse in einem flüssigen Reaktionsgemisch, das eine Base und ein gegenüber der eingesetzten Base chemisch inertes Lösungsmittel der Oligomere der beta-Hydroxybuttersäure enthält, unterzieht.

2. Verfahren nach Anspruch 1, dadurch gekennzeichnet, daß die eingesetzten Oligomere eine molekulare Masse kleiner als 500 Dalton aufweisen.

3. Verfahren nach Anspruch 1 oder 2, dadurch gekennzeichnet, daß die eingesetzten Oligomere in dem Lösungsmittel in Salz-oder Säure-Form löslich sind.

4. Verfahren nach einem der Ansprüche 1 bis 3, dadurch gekennzeichnet, daß man ein Lösungsmittel einsetzt in dem die Base löslich ist.

5. Verfahren nach einem der Ansprüche 1 bis 4, dadurch gekennzeichnet, daß das eingesetzte Lösungsmittel Wasser ist.

6. Verfahren nach einem der Ansprüche 1 bis 5, dadurch gekennzeichnet, daß man eine starke Base einsetzt.

7. Verfahren nach Anspruch 6, dadurch gekennzeichnet, daß man ein Alkalimetall- oder Erdalkalimetallhydroxyd einsetzt.

8. Verfahren nach Anspruch 7, dadurch gekennzeichnet, daß man eine Base ausgewählt unter Calciumhydroxyd, Natriumhydroxyd oder Kaliumhydroxyd einsetzt.

9. Verfahren nach einem der Ansprüche 1 bis 8, dadurch gekennzeichnet, daß das Reaktionsgemisch auf einem pH zwischen 9 und 12 gehalten wird.

10. Verfahren nach einem der Ansprüche 1 bis 9, dadurch gekennzeichnet, daß das Reaktionsgemisch auf einer Temperatur zwischen 50 und 80 °C gehalten wird.

11. Verfahren zur Herstellung von beta-Hydroxybuttersäure und ihren Salzen aus polymeren, dadurch gekennzeichnet, daß es wenigstens die folgenden Stufen umfaßt:

a) In einer ersten Stufe werden die Polymere einer Hydrolyse in einem flüssigen Reaktionsgemisch, das ein organisches Lösungsmittel, diese Polymere, einen Säure-Katalysator und Wasser enthält, bis zum Erhalt von Oligomeren, unterzogen.

b) In einer zweiten Stufe werden die in der ersten Stufe erhaltenen Oligomere einer Hydrolyse in einem flüssigen Reaktionsgemisch, das eine Base und ein gegenüber der eingesetzten Base chemisch inertes Lösungsmittel der Oligomere von beta-Hydroxybuttersäure enthält, unterzogen.